# EUROPEAN PATENT SPECIFICATION

(11) **EP 1 814 833 B1**
(45) Date of publication and mention of the grant of the patent: **12.11.2008**
(21) Application number: 05796524.6
(22) Date of filing: 09.09.2005
(51) Int. Cl.: C07C 2/28, C07C 7/13

(54) **DIISOBUTYLENE PROCESS**
DIISOBUTYLENVERFAHREN
PROCEDE DE PRODUCTION DE DIISOBUTYLENE

(30) Priority: 22.11.2004 US 994513
(43) Date of publication of application: 08.08.2007
(73) Proprietor: Lyondell Chemical Technology, L.P., Greenville, DE 19807 (US)
(72) Inventor: LIN, Shaw-Chan, West Chester, Pennsylvania 19380 (US); KAHN, Andrew, P., Eagleville, Pennsylvania 19403 (US); ZAK, Thomas, S., West Chester, Pennsylvania 19382 (US)
(74) Representative: De Hoop, Eric
(86) International application number: PCT/US2005/032332
(87) International publication number: WO 2006/057692

(56) References cited:
- EP-A- 1 034 841
- WO-A-01/46095
- WO-A-20/04085352
- US-A- 4 100 220

## Description

### FIELD OF THE INVENTION

This invention relates to a process for producing diisobutylene from isobutylene. The process comprises contacting isobutylene with a solid adsorbent to remove sulfur and/or nitrogen impurities followed by oligomerizing the isobutylene to diisobutylene using a sulfonic acid-type ion exchange resin. The solid adsorbent removes sulfur and/or nitrogen impurities from the isobutylene and surprisingly increases both catalyst activity and catalyst life of the sulfonic acid-type ion exchange resin. Optionally, the diisobutylene product may be hydrogenated to isooctane using a hydrogenation catalyst.

### BACKGROUND OF THE INVENTION

The oligomerization of olefins such as isobutylene using a sulfonic acid-type ion exchange resin catalyst is well-known in the art. For instance, U.S. Pat. No. 4,100,220 describes isobutylene oligomerization using a sulfonic acid resin catalyst and tertiary butanol selectivity enhancing modifier. In addition, U.S. Pat. No. 4,447,668 discloses isobutylene oligomerization using sulfonic acid resin catalyst A-15 with methyl t-butyl ether as solvent. Further, U.S. Pat. No. 5,877,372 describes the selective oligomerization of isobutylene using a sulfonic acid resin catalyst, tertiary butanol selectivity enhancing modifier and isooctane diluent. Lastly, U.S. Pat. No. 6,376,731 discloses the oligomerization of isobutylene in the presence of a C₃-C₄ alkane diluent to enhance oligomerization selectivity and tertiary butanol to promote selectivity to diisobutylene.

The diisobutylene product may be used as such or may be hydrogenated to isooctane as described in U.S. Pat. Nos. 5,877,372 and No. 6,376,731. Diisobutylene and isooctane are potential fuel blending compositions.

In sum, new methods to produce diisobutylene by oligomerization of isobutylene over a sulfonic acid-type ion exchange resin catalyst are needed. We have surprisingly found that sulfur and/or nitrogen impurities in the isobutylene feed lower catalyst activity and result in deactivation of the sulfonic acid-type ion exchange resin catalysts during the oligomerization step.

### SUMMARY OF THE INVENTION

This invention is a process for producing diisobutylene. The process comprises first contacting isobutylene with a solid adsorbent selected from the group consisting of molecular sieves, aluminum oxiaes, silicon oxides, silica-aluminas, sulfonic acid resins, and mixtures thereof, to remove sulfur and/or nitrogen impurities from the isobutylene, then oligomerizing the isobutylene over a sulfonic acid-type ion exchange resin catalyst to produce diisobutylene. We have found that the use of the solid adsorbent results in higher isobutylene conversion and stabilized catalyst activity.

### DETAILED DESCRIPTION OF THE INVENTION

The process of the invention comprises oligomerizing isobutylene over a sulfonic acid-type ion exchange resin catalyst to produce diisobutylene. Sulfonic acid resin catalysts are well known. Commercial examples of sulfonic acid resin catalysts include Amberlyst A-15, Amberlyst A-35, Amberlyst A-36, Purolite 275, Dowex 50 and the like. The oligomerization of isobutylene using sulfonic acid resin catalysts is well known in the art and has been described in U.S. Pat. Nos. 4,100,220, 4,447,668, 5,877,372, and 6,376,731.

The oligomerization of isobutylene typically is done by contacting the isobutylene feed with a sulfonic acid resin catalyst such as Amberlyst A-15 of Rohm & Haas, at oligomerization reaction conditions whereby exceedingly high reaction selectivity to the dimer is achieved. Generally small amounts of trimer are also formed in the oligomerization reaction. Usually, less than 10% of the converted isobutylene is converted into triisobutylene coproduct. In general, known oligomerization conditions can be employed in the oligomerization step. Suitable conditions include temperatures broadly in the range 0°C to 200°C, preferably 10°C to 100°C, and the use of pressures sufficient to maintain the liquid phase, illustratively above 50 psig, e.g. 50-500 psig.

The isobutylene feed useful in the process of the invention includes any source of isobutylene. Suitable sources of isobutylene include isobutylene-containing feedstreams, such as the refinery Cat B-B and Raffinate-1, or pure isobutylene from the dehydration of tertiary butanol as described in U.S. Pat. Nos. 5,625,109, 3,510,538, 4,165,343, and 4,155,945. The production of tertiary butyl alcohol by means of the Oxirane process is well known and widely practiced on an industrial scale, see, for example, U.S. No. Pat. 3,351,635. Cat B-B (sometimes known as Refinery B-B) feedstream is a C₄ stream (primarily butenes and butanes) obtained during the refining of crude oil by fluid catalytic cracking. Raffinate-1 is produced in the steam cracking process after the selective separation of 1,3-butadiene. Preferably, the isobutylene feed is a Cat B-B feedstream from refinery fluid catalytic cracking. The amount of isobutylene in the isobutylene feed may range from approximately 8 weight percent to essentially pure isobutylene.

During the oligomerization process, the isobutylene may be fed to the oligomerization reactor neat or in the presence of certain diluents. The presence of diluents is preferred. Tertiary butanol and/or water are preferably employed as a selectivity enhancing modifier. The use of tertiary butanol is taught in U.S. Pat. Nos No. 4,100,220, 5,877,372, and 6,376,731. In addition, the use of a C₃-C₁₀ alkane diluent is preferred in order to further enhance reaction selectivity by reducing isobutylene feed concentration, and to aid in removal of the reaction exotherm. The use of alkane diluents is taught in U.S. Pat. Nos. 5,877,372 and No. 6,376,731.

The oligomerization product contains diisobutylene as well as some unreacted isobutylene and triisobutylene coproduct. It may be necessary to separate the diisobutylene from isobutylene using conventional procedures. If isobutylene is separated from the diisobutylene product, the isobutylene may be recycled back to the oligomerization reactor.

Prior to use in olefin oligomerization, the isobutylene feed is contacted with a solid adsorbent bed to remove sulfur and/or nitrogen impurities. Removal of sulfur and/or nitrogen impurities by solid-liquid extraction methods using adsorbents is well-known in the art. In a typical extraction, the isobutylene is contacted in the liquid phase with at least one solid adsorbent. Solid adsorbents useful in the invention include any adsorbent that is capable of removing sulfur and/or nitrogen impurities from the isobutylene feed. Suitable adsorbents are molecular sieves, aluminum oxides, silicon oxides, silica-aluminas, and sulfonic acid resins such as Amberlyst 15 (available from Rohm and Haas). Molecular sieves such as 13X molecular sieves, ZSM-5, zeolite X or zeolite Y are especially preferred. Useful aluminum oxides, silicon oxides, and silica-aluminas may also include supported transition metals such as nickel. Particularly useful adsorbents include 13X molecular sieves.

The adsorptive contact is conveniently carried out at moderate temperatures, although temperature is not critical. Suitable temperatures are in the range of about 0°C to 150°C, preferably 20°C to 60°C. The flow rates are not critical, however flow rates of about 0.2 to 10 volumes of isobutylene feed per volume of adsorbent per hour are preferred, with a flow rate of about 1 to 5 volumes particularly preferred. If a sulfonic acid resin is used as the solid adsorbent, the contacting procedure is performed under conditions that do not lead to dimerization of isobutylene. It is generally preferred to employ more than one adsorbent contact beds so that a depleted bed can be regenerated while a fresh bed is used. Regeneration can be accomplished by any known means. For example, regeneration can be accomplished by simply depressurizing the bed, or by washing with water, methanol, or other solvents, followed by drying, or by stripping with a heated inert gas such as steam, nitrogen or the like.

The solid adsorbent retains the sulfur and/or nitrogen impurities adsorbed thereon resulting in isobutylene having lower sulfur and/or nitrogen impurities. Initially, there can be substantially complete removal of the sulfur and/or nitrogen impurities and the isobutylene fed to the dimerization reactor is of exceptional purity. Over the course of time the contact solids gradually become less effective for the removal of these components. Preferably, the contacting step removes at least 30 percent of the sulfur and at least 50 percent of the nitrogen content from the isobutylene. More preferably, greater than about 50 percent of the sulfur and 70 percent of the nitrogen content is removed during contacting. After contacting, the isobutylene is then separated and fed to the oligomerization reactor.

In accordance with the present invention at a pre-determined time when the separation efficiency has fallen below a desired point, the solid adsorbent is preferentially regenerated, by contact with a heated vapor stream such as nitrogen or air at a temperature of at least 200°C, or by wash with a solvent such as methanol, acetone or water, or by simply depressurizing the solid adsorbent bed. It is advantageous to employ a plurality of parallel contact zones such that while one zone is being regenerated the feed is passed through a zone containing fresh or regenerated contact material so that optimum impurities removal can be achieved.

Following isobutylene oligomerization, the diisobutylene is optionally hydrogenated to isooctane. The hydrogenation step can be carried out using conventional methods. For example, the diisobutylene may be brought into contact with hydrogen in the liquid phase at moderate temperatures and pressures. Suitable reaction temperatures vary from 0°C to 500°C, but preferably from 25°C to 200°C. The reaction is preferably conducted at or above atmospheric pressure. The precise pressure is not critical. Typical pressures vary from 1 atmosphere to 100 atmospheres. Any suitable hydrogenation catalyst may be used, including but not limited to Raney nickel and supported nickel, palladium, and platinum catalysts. Suitable supports for nickel, palladium, and platinum include carbon, silica, alumina, diatomaceous earth, and the like. The hydrogenation may be performed in the presence or absence of a solvent. Following hydrogenation, the isooctane product can be recovered by removing the hydrogenation catalyst and the solvent (if present) in a conventional manner, to separate isooctane.

The hydrogenation reaction may be performed using any of the conventional reactor configurations known in the art for such hydrogenation processes. Continuous as well as batch procedures may be used. For example, the catalyst may be deployed in the form of a fixed bed or slurry.

The following examples merely illustrate the invention.

### COMPARATIVE EXAMPLE 1: ISOBUTYLENE DIMERIZATION USING NON-TREATED ISOBUTYLENE

An isobutylene reaction feed comprising Cat B-B refinery isobutylene (95 wt.%) and tertiary butyl alcohol (TBA, 5 wt.%) is fed (at 63 grams/hour) to a 1" fixed bed reactor containing sulfonated ion exchange resin (30 g, Purolite CT275, WHSV = 2.1 h⁻¹, 93°C). The flow rate of the isobutylene reaction feed is 63 grams/hour (2.1 WHSV). The Cat B-B refinery isobutylene contains 16.78 wt.% isobutylene, 28.37 wt.% isobutane, 13.55 wt.% n-butane, 13.07 wt.% 1-butene, 0.33 wt.% 1,3-butadiene, 14.26 wt.% trans 2-butene, 10.37 wt.% cis 2-butene, 1.17 wt.% propane, 1.41 wt.% propylene, 0.69 wt.% 1-pentene, 15 ppm S, and 65 ppm N. The reactor is maintained at 93°C using a three-zone furnace and the product stream is analyzed by GC at various times following reaction start-up.

### EXAMPLE 2: ISOBUTYLENE DIMERIZATION USING NON-TREATED ISOBUTYLENE

The procedure of example 1 is repeated, except that the isobutylene reaction feed comprising Cat B-B refinery isobutylene (95 wt.%) and tertiary butyl alcohol (TBA, 5 wt.%) is first fed to a 1" stainless tube charged with 90 grams of UOP 13X HP Molecular Sieve absorbent prior to oligomerization at 22°C. The feed (which contained 8-10 ppm S and 11 ppm N following contact with adsorbent) is passed continuously through the 13X molecular sieve adsorbent and then into the oligomerization reactor.

The results, shown in Table 1, demonstrate that the oligomerization catalyst performs much better (higher isobutylene conversion) and are more stable (steady conversion) than the corresponding test without the solid adsorbent.

**TABLE 1: ISOBUTYLENE DIMERIZATION RESULTS.**

| Comparative Example 1 | | Example 2 | |
|---|---|---|---|
| Hours on Stream | Isobutylene Conversion (%) | Hours on Stream | Isobutylene Conversion (%) |
| 127 | 24.5 | 96 | 30.9 |
| 131 | 22.7 | 116 | 31.6 |
| 151 | 16.0 | 130 | 31.2 |
| 167 | 11.7 | 142 | 31.6 |

## Claims

1. A process comprising:
(a) contacting an isobutylene feedstream containing sulfur and/or nitrogen impurities with a solid adsorbent to produce a contacted isobutylene having a reduced amount of sulfur and/or nitrogen impurities; wherein the solid adsorbent is selected from the group consisting of molecular sieves, aluminum oxides, silicon oxides, silica-aluminas, sulfonic acid resins, and mixtures thereof; and
(b) oligomerizing the contacted isobutylene in the presence of a sulfonic acid resin catalyst to produce diisobutylene.

2. The process of claim 1 wherein the solid adsorbent is selected from the group consisting of 13X molecular sieves, ZSM-5, zeolite X, zeolite Y, and mixtures thereof.

3. The process of claim 1 wherein the solid adsorbent comprises 13X molecular sieves.

4. The process of claim 1 wherein the isobutylene feedstream is a Cat B-B feedstream.

5. The process of claim 1 further comprising hydrogenating the diisobutylene in the presence of a hydrogenation catalyst to form isooctane.

6. The process of claim 5 wherein the hydrogenation catalyst is a supported nickel catalyst.

7. The process of claim 5 wherein the solid adsorbent is selected from the group consisting of 13X molecular sieves, ZSM-5, zeolite X, zeolite Y, and mixtures thereof.

8. The process of claim 5 wherein the solid adsorbent comprises 13X molecular sieves.

9. The process of claim 5 wherein the isobutylene feedstream is a Cat B-B feedstream.

## Patentansprüche

1. Verfahren, umfassend:
(a) Kontaktieren eines Isobutylen-Zufuhrstroms, der Schwefel- und/oder Stickstoff-Verunreinigungen enthält, mit einem festen Adsorbens zur Herstellung eines kontaktierten Isobutylens, das eine reduzierte Menge an Schwefel- und/oder Stickstoff-Verunreinigungen aufweist; wobei das feste Adsorbens aus der Gruppe ausgewählt wird, die aus Molekularsieben, Aluminiumoxiden, Siliziumoxiden, Siliziumdioxid-Aluminiumoxiden, Sulfonsäureharzen und Mischungen daraus besteht; und
(b) Oligomerisieren des kontaktierten Isobutylens bei Vorhandensein eines Sulfonsäureharz-Katalysators zur Herstellung von Diisobutylen.

2. Verfahren nach Anspruch 1, bei dem das feste Adsorbens aus der Gruppe ausgewählt wird, die aus 13X-Molekularsieben, ZSM-5, Zeolith X, Zeolith Y und Mischungen daraus besteht.

3. Verfahren nach Anspruch 1, bei dem das feste Adsorbens 13X-Molekularsiebe umfasst.

4. Verfahren nach Anspruch 1, bei dem der Isobutylen-Zufuhrstrom ein Cat-B-B-Zufuhrstrom ist.

5. Verfahren nach Anspruch 1, das weiterhin die Hydrierung des Diisobutylen bei Vorhandensein eines Hydrierungs-Katalysators zur Bildung von Isooktan umfasst.

6. Verfahren nach Anspruch 5, bei dem der Hydrierungs-Katalysator ein NickelTrägerkatalysator ist.

7. Verfahren nach Anspruch 5, bei dem das feste Adsorbens aus der Gruppe ausgewählt wird, die aus 13X-Molekularsieben, ZSM-5, Zeolith X, Zeolith Y und Mischungen daraus besteht.

8. Verfahren nach Anspruch 5, bei dem das feste Adsorbens 13X-Molekularsiebe umfasst.

9. Verfahren nach Anspruch 5, bei dem der Isobutylen-Zufuhrstrom ein Cat-B-B-Zufuhrstrom ist.

## Revendications

1. Procédé comprenant :
(a) le contact d'un courant d'alimentation d'isobutylène contenant des impuretés soufrées et/ou azotées avec un adsorbant solide pour produire un isobutylène contacté ayant une quantité réduite d'impuretés soufrées et/ou azotées ; dans lequel l'adsorbant solide est choisi dans le groupe comprenant les tamis moléculaires, les oxydes d'aluminium, les oxydes de silicium, les silice-alumines, les résines d'acide sulfonique et les mélanges des absorbants précédents ; et
(b) l'oligomérisation de l'isobutylène contacté en présence d'un catalyseur à résine d'acide sulfonique pour produire du diisobutylène.

2. Procédé selon la revendication 1, dans lequel l'adsorbant solide est choisi dans le groupe comprenant les tamis moléculaires 13X, la ZSM-5, la zéolithe X, la zéolithe Y est leurs mélanges.

3. Procédé selon la revendication 1, dans lequel l'adsorbant solide comprend des tamis moléculaires 13X.

4. Procédé selon la revendication 1, dans lequel le courant d'alimentation d'isobutylène est un courant d'alimentation Cat B-B.

5. Procédé selon la revendication 1, comprenant en outre l'hydrogénation du diisobutylène en présence d'un catalyseur d'hydrogénation pour former de l'isooctane.

6. Procédé selon la revendication 5, dans lequel le catalyseur d'hydrogénation est un catalyseur à base de nickel supporté.

7. Procédé selon la revendication 5, dans lequel l'adsorbant solide est choisi dans le groupe comprenant les tamis moléculaires 13X, la ZSM-5, la zéolithe X, la zéolithe Y est leurs mélanges.

8. Procédé selon la revendication 5, dans lequel l'adsorbant solide comprend des tamis moléculaires 13X.

9. Procédé selon la revendication 5, dans lequel le courant d'alimentation d'isobutylène est un courant d'alimentation Cat B-B.
